# EUROPEAN PATENT SPECIFICATION

(11) **EP 4 335 862 B1**
(45) Date of publication and mention of the grant of the patent: **27.08.2025**
(21) Application number: 22864983.6
(22) Date of filing: 29.08.2022
(51) Int. Cl.: A61K 38/17, C12N 9/00, A61P 35/00, C07H 19/207, C12N 9/10

(54) **ADP-RIBOSE BINDING PEPTIDE HAVING ANTICANCER ACTIVITY AND USE THEREOF**
ADP-RIBOSE-BINDENDES PEPTID MIT ANTIKREBSWIRKUNG UND VERWENDUNG DAVON
PEPTIDE DE LIAISON À L'ADP-RIBOSE AYANT UNE ACTIVITÉ ANTICANCÉREUSE ET UTILISATION ASSOCIÉE

(30) Priority: 02.09.2021 KR 20210117046
(43) Date of publication of application: 13.03.2024
(73) Proprietor: Pearlsinmires Co., Ltd., Seoul 07292 (KR)
(72) Inventor: JEONG, Keun-Yeong, Seoul 03690 (KR); PARK, Min Hee, Siheung-si, Gyeonggi-do 14914 (KR)
(74) Representative: HGF
(86) International application number: PCT/KR2022/012886
(87) International publication number: WO 2023/033481

(56) References cited:
- WO-A2-2015/191546
- US-A1- 2020 384 118
- WANG ZHIZHI ET AL: "Recognition of the iso-ADP-ribose moiety in poly(ADP-ribose) by WWE domains suggests a general mechanism for poly(ADP-ribosyl)ation-dependent ubiquitination", vol. 26, no. 3, 1 February 2012 (2012-02-01), US, pages 235 - 240, XP093042868, ISSN: 0890-9369, Retrieved from the Internet <URL:https://www.ncbi.nlm.nih.gov/pmc/articles/PMC3278890/pdf/235.pdf> DOI: 10.1101/gad.182618.111
- ARAVIND L: "The WWE domain: a common interaction module in protein ubiquitination and ADP ribosylation", TRENDS IN BIOCHEMICAL SCIENCES, ELSEVIER, AMSTERDAM, NL, vol. 26, no. 5, 1 May 2001 (2001-05-01), pages 273 - 275, XP004241859, ISSN: 0968-0004, DOI: 10.1016/S0968-0004(01)01787-X
- WANG ZHIZHI, MICHAUD GREGORY A., CHENG ZHIHONG, ZHANG YUE, HINDS THOMAS R., FAN ERKANG, CONG FENG, XU WENQING: "Recognition of the iso-ADP-ribose moiety in poly(ADP-ribose) by WWE domains suggests a general mechanism for poly(ADP-ribosyl)ation-dependent ubiquitination", GENES & DEVELOPMENT, COLD SPRING HARBOR LABORATORY PRESS, PLAINVIEW, NY., US, vol. 26, no. 3, 1 February 2012 (2012-02-01), US , pages 235 - 240, XP093042868, ISSN: 0890-9369, DOI: 10.1101/gad.182618.111
- KOO JA-HYUN, YOON HEESEOK, KIM WON-JU, CHA DONGHUN, CHOI JE-MIN: "Cell-Penetrating Function of the Poly(ADP-Ribose) (PAR)-Binding Motif Derived from the PAR-Dependent E3 Ubiquitin Ligase Iduna", INTERNATIONAL JOURNAL OF MOLECULAR SCIENCES, vol. 19, no. 3, 8 March 2018 (2018-03-08), pages 779, XP093042870, DOI: 10.3390/ijms19030779
- KRIETSCH JANA, ROULEAU MICHÈLE, PIC ÉMILIE, ETHIER CHANTAL, DAWSON TED M., DAWSON VALINA L., MASSON JEAN-YVES, POIRIER GUY G., GAG: "Reprogramming cellular events by poly(ADP-ribose)-binding proteins", MOLECULAR ASPECTS OF MEDICINE, PERGAMON PRESS, OXFORD., GB, vol. 34, no. 6, 1 December 2013 (2013-12-01), GB , pages 1066 - 1087, XP093042872, ISSN: 0098-2997, DOI: 10.1016/j.mam.2012.12.005
- PALAZZO LUCA, AHEL IVAN: "PARPs in genome stability and signal transduction: implications for cancer therapy", BIOCHEMICAL SOCIETY TRANSACTIONS, PORTLAND PRESS LTD, GB, vol. 46, no. 6, 17 December 2018 (2018-12-17), GB , pages 1681 - 1695, XP093042873, ISSN: 0300-5127, DOI: 10.1042/BST20180418

## Description

### [Technical Field]

The present disclosure relates to an adenosine diphosphate (ADP)-ribose binding peptide with anti-cancer activity, and specifically, an ADP-ribose binding peptide having a specific amino acid sequence and a modification thereof, a pharmaceutical composition for the prevention or treatment of cancer comprising the same as an active ingredient, and a pharmaceutical composition for anti-cancer adjuvant.

### [Background Art]

Poly ADP-ribosylation (PARylation) is one of the posttranslation modification processes, in which ADP-ribose polymer (poly(adenosine diphosphate-ribose)) is covalently attached to a protein by PAR polymerase. The PARylation produces a polymer ADP-ribose chain, and may induce a unique intracellular biochemical action that is not a small molecular level modification such as acetylation or methylation and does not show a form such as ubiquitination or SUMOylation. The balance of PARylation plays an important role in DNA damage repair, regulation of transcription, change in chromatin structure, oxidative/reduction homeostasis, transduction of various intracellular signals, formation of non-membrane structures, host-pathogen interactions, and regulation of RNA metabolism (Juan et al., Cancers, 2020, 12(3): 739).

PARylation has been implicated in the pathogenesis of systemic diseases including cancer, viral infections, and neurodegeneration. In particular, since PARylation is derived from the activation of poly [ADP-ribose] polymerase 1 (PARP-1), the anti-cancer effects of targeting ovarian cancer, prostate cancer, breast cancer, and other cancers by methods of inhibiting the activity of PARP-1 are already well known (J Mateo et al., Ann Oncol., 2019, 30(9): 1437).

Information on the biochemical mechanisms involved in PARylation-mediated cell death has been known recently, and activation of PARylation may typically induce cell death through three main pathways (Rebecca Gupte et al., Genes Dev. 2017, 31(2): 101).

Depletion of nicotinamide adenine dinucleotide (NAD⁺) may impair cellular metabolism that occurs in a cellular energy crisis (ATP depletion), especially an oxidative phosphorylation process. It has been suggested that PARylation activation induced by extensive DNA damage consumes NAD⁺, which may result in cell death as a subsequent effect of this consuming action.

In addition, PAR polymers released from the nucleus after DNA repair may liberate cell death-inducing factors bound to the mitochondrial membrane in the cytoplasm and activate pathways that mobilize cell death-inducing factors into the nucleus. When cell death factors translocate to the nucleus, large-scale DNA fragmentation is mediated and induces cell death. PARylation-induced energy depletion and cell death-inducing factors are known to be involved in PAR signal transduction that controls protein kinase-phosphatase pathways such as the PI3K-Akt pathway or the MAP kinase pathway, and this PAR-dependent cell death process contributes to a significant extent of complexity of the death mechanism.

Further, it has been reported that autoPARylation of PARP-1 is able to degrade PARP-1 itself and induce cell death even though a clear biochemical mechanism thereof has not yet been identified. However, cancer may evade this death in a clever way by activating several proteolytic enzymes to prevent the death process, thereby degrading surplus PAR polymers involved in various biochemical processes necessary for cancer survival.

Therefore, contrary to the conventional attempts to inhibit PARylation, such as PARP-1 inhibitors, activating PARylation or inhibiting the degradation of PAR polymers may also be one strategy for discovering effective anti-cancer therapies. In particular, activation of PARylation or inhibition of PAR polymer degradation may be considered a very promising target in cancer cell-specific regions that undergo metabolic processes in a different way from that of normal cells.

### [Disclosure]

### [Technical Problem]

The present inventors made great efforts to develop a novel anti-cancer drug, and as a result, confirmed that peptides having the novel amino acid sequence of the present disclosure could bind to ADP-ribose and ultimately prevent the utilization of ADP-ribose or PAR polymer from the action of various degrading enzymes or signaling proteins in cells, thereby overactivating PARylation and disturbing the degradation process of PAR polymerase, and this disturbance could induce the death of cancer cells, resulting in revolutionary anti-cancer efficacy, and completed the present disclosure.

### [Technical Solution]

An object of the present disclosure is to provide an adenosine diphosphate (ADP)-ribose binding peptide having any one amino acid sequence selected from the group consisting of SEQ ID NOs: 1 to 14.

Another object of the present disclosure is to provide a polynucleotide encoding the ADP-ribose binding peptide as described above.

Still another object of the present disclosure is to provide a vector and a transformant comprising the polynucleotide as described above.

Still another object of the present disclosure is to provide a pharmaceutical composition for preventing or treating cancer, a pharmaceutical composition for anti-cancer adjuvants to enhance reactivity to a second anti-cancer drug, and a pharmaceutical composition for anti-cancer adjuvants to enhance reactivity to anti-cancer radiation therapy, each composition comprising: the ADP-ribose binding peptide as described above or a pharmaceutically acceptable salt thereof as an active ingredient.

Still another object of the present disclosure is to provide use of the peptide or a pharmaceutically acceptable salt thereof for the prevention or treatment of cancer, and a method for preventing or treating cancer comprising administering the peptide or a pharmaceutically acceptable salt thereof to a subject in need thereof.

### [Advantageous Effects]

The ADP-ribose binding peptide of the present disclosure has an excellent anti-cancer effect by accumulating ADP-ribose in cancer cells to destroy the balance, thus leading to the death of cancer cells, but not being toxic to normal cells, and when administered in combination with other anti-cancer drugs or anti-cancer radiation therapy, has a very good effect as an anti-cancer adjuvant by enhancing the reactivity to the anti-cancer drugs and radiation therapy.

### [Description of Drawings]

FIG. 1 shows the level of intracellular poly ADP-ribose after treating different cancer cells with different concentrations of peptides of SEQ ID NOs: 1 to 4.
FIG. 2 shows the level of intracellular poly ADP-ribose after treating different cancer cells with different concentrations of peptides of SEQ ID NOs: 5 to 8.
FIG. 3 shows the level of intracellular poly ADP-ribose after treating different cancer cells with different concentrations of peptides of SEQ ID NOs: 9 to 12.
FIG. 4 shows the level of intracellular poly ADP-ribose after treating different cancer cells with different concentrations of peptides of SEQ ID NOs: 13 and 14.
FIG. 5 shows the results of confirming the cell viability after treating different cancer cells with the peptides of SEQ ID NO: 1, SEQ ID NO: 15, SEQ ID NO: 2, and SEQ ID NO: 16, in which the top images showed cells, and the bottom graphs showed cell viability.
FIG. 6 shows the result of confirming the cell viability after treating different cancer cells with the peptides of SEQ ID NO: 3, SEQ ID NO: 17, SEQ ID NO: 4, and SEQ ID NO: 18, in which the top images showed cells, and the bottom graphs showed cell viability.
FIG. 7 shows the result of confirming the cell viability after treating different cancer cells with the peptides of SEQ ID NO: 5, SEQ ID NO: 19, SEQ ID NO: 6, and SEQ ID NO: 20, in which the top images showed cells, and the bottom graphs showed cell viability.
FIG. 8 shows the result of confirming the cell viability after treating different cancer cells with the peptides of SEQ ID NO: 7, SEQ ID NO: 21, SEQ ID NO: 8, and SEQ ID NO: 22, in which the top images showed cells, and the bottom graphs showed cell viability.
FIG. 9 shows the result of confirming the cell viability after treating different cancer cells with the peptides of SEQ ID NO: 9, SEQ ID NO: 23, SEQ ID NO: 10, and SEQ ID NO: 24, in which the top images showed cells, and the bottom graphs showed cell viability.
FIG. 10 shows the result of confirming the cell viability after treating different cancer cells with the peptides of SEQ ID NO: 11, SEQ ID NO: 25, SEQ ID NO: 12, and SEQ ID NO: 26, in which the top images showed cells, and the bottom graphs showed cell viability.
FIG. 11 shows the result of confirming the cell viability after treating different cancer cells with the peptides of SEQ ID NO: 13, SEQ ID NO: 27, SEQ ID NO: 14, and SEQ ID NO: 28, in which the top images showed cells, and the bottom graphs showed cell viability.
FIG. 12 shows the result of confirming the cell viability after treating different cancer cells with the peptides of SEQ ID NOs: 15 to 28.
FIG. 13 shows the cell viability after treatment of different cancer cells with bevacizumab (left) or osimertinib (right) alone or in combination with the peptides of SEQ ID NOs: 1 to 14.
FIG. 14 shows cell viability after treatment of different cancer cells with gemcitabine (left) or docetaxel (right) alone or in combination with the peptides of SEQ ID NOs: 15 to 28.
FIG. 15 shows the cell viability after treatment of different cancer cells with 2 Gy of radiation alone or in combination with the peptides of SEQ ID NOs: 1 to 14.
FIG. 16 shows the cell viability after treatment of different cancer cells with 2 Gy of radiation alone or in combination with the peptides of SEQ ID NOs: 15 to 28.
FIG. 17 shows a change in tumor volume over time after subcutaneous administration of the peptides of SEQ ID NOs: 15 to 28 in a tumor graft animal model.
FIG. 18 is an image of tumor tissue confirmed after subcutaneous administration of the peptides of SEQ ID NOs: 15 to 28 in the tumor graft animal model.
FIG. 19 shows a change in tumor volume over time after oral administration of the peptides of SEQ ID NOs: 1 to 14 in the tumor graft animal model.
FIG. 20 is an image of tumor tissue confirmed after oral administration of the peptides of SEQ ID NOs: 1 to 14 in the tumor graft animal model.
FIG. 21 shows the results of evaluating the toxicity to normal cells (CCD-18Co) of the peptide of SEQ ID NO: 7 used as a representative peptide of the present disclosure.
FIG. 22 shows the results of evaluating the toxicity to normal cells (HDPC) of the peptide of SEQ ID NO: 7 used as a representative peptide of the present disclosure.

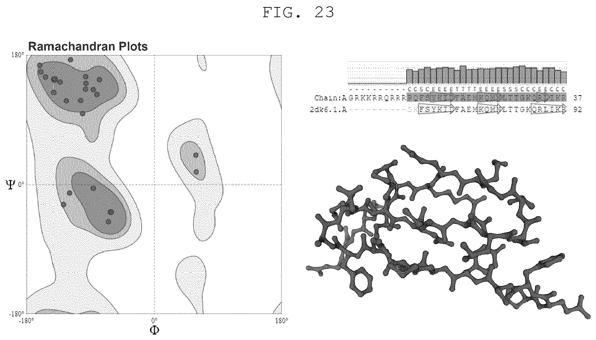

FIG. 23 shows the structure of the peptide of SEQ ID NO: 7.

### [Best Mode]

The present disclosure will be described in detail as follows. Meanwhile, each description and embodiment disclosed in the present disclosure may be applied to each of the other descriptions and embodiments. In other words, all combinations of various elements disclosed in the present disclosure fall within the scope of the present disclosure. In addition, it cannot be considered that the scope of the present disclosure is limited by specific descriptions described below, but by the claims.

An embodiment of the present disclosure for achieving the above object is an adenosine diphosphate (ADP)-ribose binding peptide having any one amino acid sequence selected from the group consisting of SEQ ID NOs: 1 to 14.

As used herein, the term "ADP-ribose" is used as a concept including both isolated ADP-ribose itself and ADP-ribose polymer (poly ADP-ribose), and the "ADP-ribose binding peptide" refers to any peptide with an activity of binding to ADP-ribose or an ADP-ribose polymer and inhibiting degradation. The present disclosure includes not simply peptides formed by peptide bonds between amino acids constituting the peptides, but also modifications of all forms of peptide analogs, derivatives, and the like, having some modified forms to improve properties such as stability, efficacy, and the like, from the viewpoint of protein drugs.

In a completely different aspect from the numerous reports related to anti-cancer efficacy obtained when PARylation (poly ADP-ribosylation) is inhibited by conventional PARP-1 inhibitors, the present inventors paid attention to the fact that anti-cancer effects could be expected even through activation of PARylation or inhibition of PAR polymer degradation. In particular, since cancer cells continue to divide at a faster rate than normal cells and actively metabolize, it was expected that by overactivating PARylation or inhibiting the degradation of PAR polymer, the cancer cells could be specifically killed without any particular effect on normal cells that did not overactivate PARylation.

All of the adenosine diphosphate (ADP)-ribose binding peptides having any one amino acid sequence selected from the group consisting of SEQ ID NOs: 1 to 14 (Table 1) of the present disclosure were derived from the WWE domain. The WWE domain is a globular domain conserved in many proteins, including deltex, Trip12, and poly-ADP-ribose polymerase homologs, and the like, named after the most conserved residue in the domain (L. Aravind, TRENDS in Biochemical Sciences, 2001, 26(5): 273). It is known that in some proteins having the WWE domain, there is an ADP-ribose binding motif in the domain. It has been reported that the WWE domain of intracellular enzymes mainly binds to ADP-ribose to induce degradation. In other words, binding of a specific enzyme to PAR or ADPR through the WWE domain may be considered as a great purpose for cancer cell survival (PNAS August 23, 2011 108 (34) 14103-14108).

However, contrary to the above, it has not been reported at all that when a portion of the WWE domain is prepared as an isolated peptide and treated with cells, ADP-ribose degradation is inhibited and ADP-ribose is accumulated in cells, thus providing excellent anti-cancer effects as in the present disclosure.

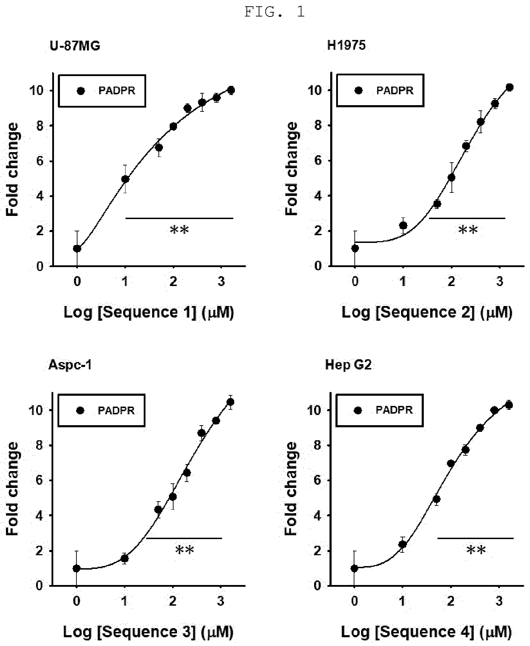

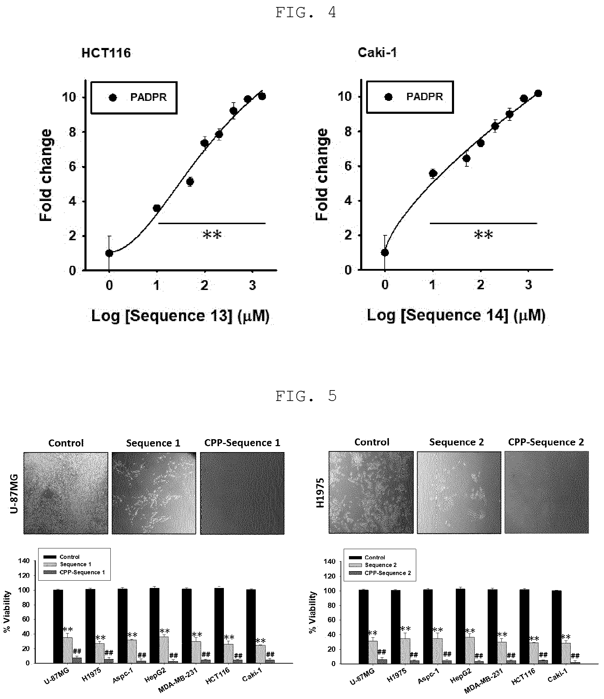

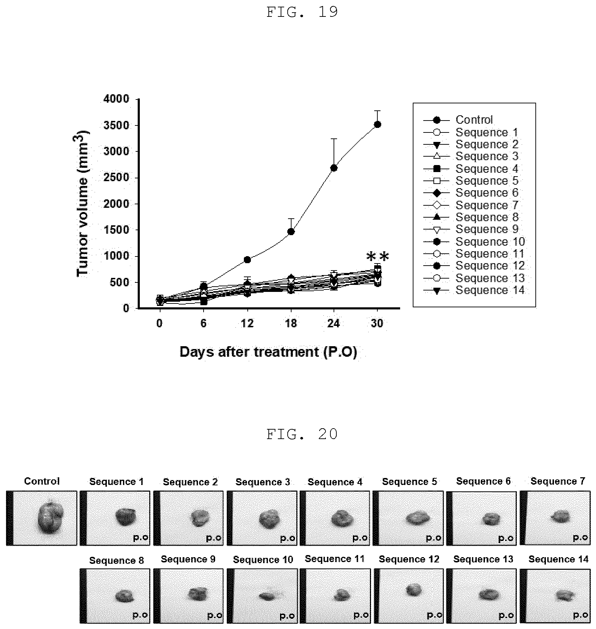

In a specific embodiment of the present disclosure, fragments were synthesized from WWE domains present in various types of proteins and an anti-cancer activity thereof was investigated. As a result, it was confirmed that when various types of cancer cells were treated with the ADP-ribose binding peptides (SEQ ID NOs: 1 to 14) of Examples 1 to 14, the level of ADP-ribose in cancer cells significantly increased (FIGS. 1 to 4). Further, it was confirmed through in vitro experiments that the treatment of the peptides disrupted the intracellular balance of ADP-ribose, such that the growth of cancer cells was suppressed and most cancer cells were killed (FIGS. 5 to 11), and also confirmed in tumor graft animal models that when the ADP-ribose binding peptides of Examples 1 to 14 were subcutaneously or orally administered, growth of tumor tissue in vivo was rapidly reduced (FIG. 19 and FIG. 20).

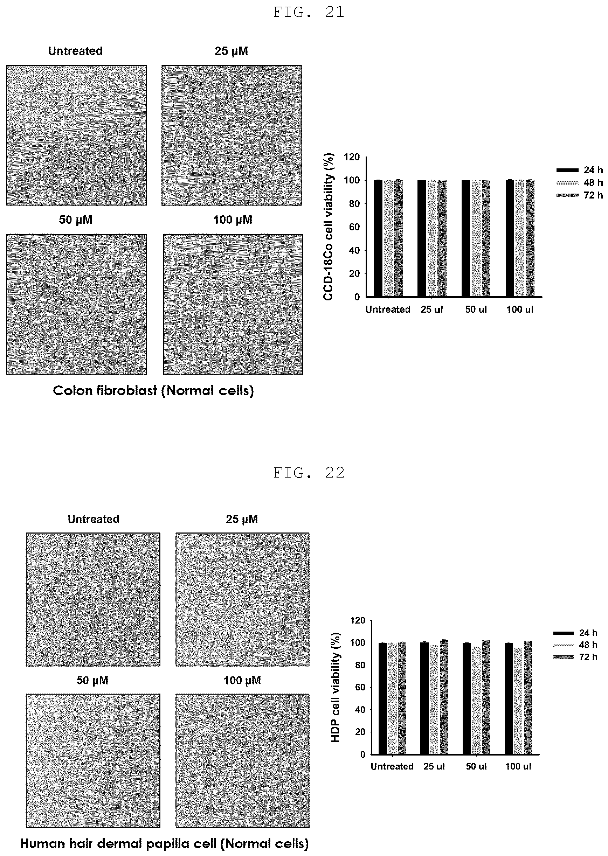

Further, in another specific embodiment of the present disclosure, normal cells were treated with a peptide of SEQ ID NO: 7 used as a representative peptide and cytotoxicity against the normal cells was evaluated. As a result, it was confirmed that no toxicity was shown in normal cells (FIGS. 21 and 22).

Therefore, the ADP-ribose binding peptides of SEQ ID NOs: 1 to 14 of the present disclosure have excellent anti-cancer effects regardless of cancer types and do not show any cytotoxicity to normal cells, which may be usefully employed as a composition for preventing or treating cancer.

All of the ADP-ribose binding peptides belong to the scope of the present disclosure as long as not only peptides having the amino acid sequence of any one of SEQ ID NOs: 1 to 14, but also peptides having sequences in which one or more amino acids are added, substituted or deleted from these sequences fall within the equivalent range.

For example, it is obvious that if a peptide has at least 80% or more, 90% or more, 95% or more, 97% or more, or 99% or more homology to a peptide having any one amino acid sequence of SEQ ID NOs: 1 to 14 of the present disclosure and exhibits an efficacy corresponding to that of the peptide comprising any one of amino acid sequences of Nos. 1 to 14, that is, ADP-ribose binding activity and anti-cancer activity, even if the peptide has an amino acid sequence in which some sequences are added, substituted, or deleted from the amino acid sequences of SEQ ID NOs: 1 to 14, the peptide is included within the scope of the present disclosure.

In addition, if the peptide has an activity corresponding to the peptide consisting of any one of the amino acid sequences of SEQ ID NOs: 1 to 14, mutations that may add meaningless sequences before and after the amino acid sequence or that may occur naturally, or silent mutations thereof may also be included within the scope of the present disclosure.

As used herein, the term "homology" refers to a degree of matching with a given amino acid sequence or base sequence, and the homology may be expressed as a percentage. In the present specification, a homology sequence having an activity that is identical or similar to the given amino acid sequence or base sequence is expressed as "% homology". For example, the homology may be confirmed using standard software, specifically BLAST 2.0, which calculates parameters such as score, identity and similarity, or by comparing sequences through Southern hybridization experiments under defined stringent conditions, wherein the appropriate hybridization conditions to be defined are within the scope of the technology and may be determined by methods well known to those skilled in the art (for example, J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York) .

In addition, the peptides of the present disclosure may include modifications such as chemical derivatization, and the like, in one or more amino acids constituting the peptides to further improve desired properties.

The derivatization may include, but is not limited to, acetylation, hydroxylation, methylation, amidation, pegylation, and the addition of carbohydrate or lipid components, carriers, and the like.

As a specific aspect of the present disclosure, the peptide may be additionally used in a fused form with a cell-penetrating peptide to increase cell-penetrating ability. In other words, the ADP-ribose binding peptide may further include cell-penetrating peptides at the N-terminus, C-terminus, or both termini. Here, a linker may be additionally included between the ADP-ribose binding peptide and the cell-penetrating peptide, which may be appropriately performed by those skilled in the art.

As used herein, the term "cell-penetrating peptide" refers to a peptide having characteristics capable of promoting uptake/absorption into cells of various materials such as nanoparticles, compounds, DNA, proteins, and the like. Specifically, the cell-penetrating peptide may include, but is not limited to, TAT, maurocalcine, penetratin, a poly-arginine-derived peptide, antennapedia, transportan, VP22, Hph-1, poly-arginine, R11(R9), Pep-1, HP4, LAH4, vetofusing-1, a signal sequence-based peptide, or an amphipathic peptide, and may be appropriately selected by those skilled in the art as long as the cell-penetrating peptide is capable of promoting the intracellular movement of the ADP-ribose binding peptide of the present disclosure.

In a specific embodiment of the present disclosure, TAT, a cell-penetrating peptide, was fused to the N-terminus of the ADP-ribose binding peptides of SEQ ID NOs: 1 to 14 of the present disclosure to evaluate the anti-cancer activity thereof (SEQ ID NOs: 15 to 24, Table 2). As a result, it could be confirmed that the fusion had more excellent anti-cancer activity than that of the ADP-ribose binding peptide alone (FIGS. 5 to 12, 17 and 18). Therefore, not only the ADP-ribose binding peptides of SEQ ID NOs: 1 to 14 of the present disclosure, but also the cell-penetrating protein fused with these ADP-ribose binding peptides may be very usefully employed as a composition for preventing or treating cancer.

Further, those skilled in the art may appropriately modify and use the ADP-ribose binding peptide of the present disclosure to be applied according to the type of cell-penetrating peptide used. In other words, even when the ADP-ribose binding peptide of the present disclosure is fused to the cell-penetrating peptide to be used, it is not limited to the amino acid sequence presented in the present disclosure, but it is possible to use amino acid sequences added/substituted/removed in a form suitable for application of the cell-penetrating peptide within the range obvious to those skilled in the art, i.e., the equivalent range.

In addition, the ADP-ribose binding peptide of the present disclosure may be employed together with reagents known in the art that are capable of delivering proteins into cells or improving delivery efficiency in order to increase cell-penetrating ability. The reagent may include, for example, not only commercially available reagents such as Chariot^{™} (Active motif, Cat. 30025), Xflect^{™} (Takara, Cat. 631324), Pierce^{™} (ThermoFisher Scientific, Cat. 89850), ProteoJuice^{™} (Merck, Cat. 71281), PULSinTM (Poylplus transfection), and the like, but also other non-commercial reagents, and is not particularly limited to the above-described examples as long as the ADP-ribose binding peptide of the present disclosure is able to be delivered to cells.

Another aspect of the present disclosure is a polynucleotide encoding the ADP-ribose binding peptide as described above.

Still another aspect of the present disclosure is a vector comprising the polynucleotide as described above.

Still another aspect of the present disclosure is a transformant comprising the polynucleotide as described above.

The ADP-ribose binding peptide is the same as described above.

The polynucleotide may have a nucleotide sequence encoding the ADP-ribose binding peptide of the present disclosure, or a nucleotide sequence having at least 80% or more, 90% or more, 95% or more, 97% or more, or 99% or more homology thereto, and if any polypeptide translated therefrom exhibits an efficacy corresponding to that of the ADP-ribose binding peptide of the present disclosure, any case where a meaningless sequence is added, or a partial sequence is deleted, modified, or substituted at the 5'-and/or 3'-terminal of the nucleotide sequence may also be included in the scope of the present disclosure. The polynucleotide may be used in the form of an expression cassette operably linked to a known promoter sequence or a vector comprising the polynucleotide, and the polynucleotide, expression cassette, or vector may be prepared appropriately by a method known to those skilled in the art. The type of promoter and vector is not particularly limited and may be appropriately selected by those skilled in the art according to the purpose. In addition, a transformant may be prepared to be used by transforming the polynucleotide, expression cassette, or vector into a host cell, and the transformation methods to be employed may also be performed without limitation by methods known to those skilled in the art. The transformant, which is subject to express the ADP-ribose binding peptide of the present disclosure, may be a microorganism, plant or animal, and may be non-human, but is not limited thereto.

Those skilled in the art may prepare and use a polynucleotide encoding the ADP-ribose binding peptide as described above, a vector comprising the same, or a transformant comprising the vector in order to apply/produce the ADP-ribose binding peptide for various purposes. For example, the polynucleotide or the vector may be used directly for the treatment of cancer, and the transformant expressing the ADP-ribose binding peptide including the polynucleotide or the vector may be used to produce the peptide or used for therapeutic purposes, but is not limited thereto.

Still another aspect of the present disclosure is a pharmaceutical composition for preventing or treating cancer, comprising the ADP-ribose binding peptide as described above or a pharmaceutically acceptable salt thereof as an active ingredient.

Still another aspect of the present disclosure is the use of the peptide as described above or a pharmaceutically acceptable salt thereof for the prevention or treatment of cancer, and a method for preventing or treating cancer comprising administering the peptide or a pharmaceutically acceptable salt thereof to a subject in need thereof.

As described above, the ADP-ribose binding peptide of the present disclosure has an excellent effect in preventing and/or treating cancer.

In the present disclosure, the term "cancer" refers to a disease related to the regulation of cell death, and refers to a disease caused by excessive proliferation of cells when the normal balance of cell death is broken. As used herein, cancer includes both malignant and benign tumors, and may be, for example, brain cancer, head and neck cancer, lung cancer, breast cancer, thymoma, esophageal cancer, colon cancer, liver cancer, gastric cancer, pancreatic cancer, bile duct cancer, kidney cancer, bladder cancer, prostate cancer, testicular cancer, germ cell tumor, ovarian cancer, cervical cancer, endometrial cancer, colorectal cancer, lymphoma, acute leukemia, chronic leukemia, multiple myeloma, sarcoma, malignant melanoma or skin cancer, but the types of cancer of the present disclosure are not limited by the above examples. The composition for preventing or treating cancer of the present disclosure has a therapeutic effect on all cancers in which ADP-ribose accumulates in cells, resulting in cell death.

For an example, cancer may be solid tumors such as brain cancer, lung cancer, pancreatic cancer, liver cancer, breast cancer, colon cancer, kidney cancer, gastric cancer, or ovarian cancer, but is not limited thereto.

As used herein, the term "treatment" refers to intervention to alter the natural processes of individuals or cells having diseases, which may be performed during the progression of pathology or to prevent the pathology. Desired therapeutic effects include prevention of the occurrence or recurrence of the disease, alleviation of symptoms, reduction of any direct or indirect pathological consequences of the disease, prevention of metastasis, a decrease of the rate of disease progression, reduction or temporary alleviation of the disease state, and remission or improvement of prognosis. In particular, the present disclosure includes all actions that delay the progress of cancer by administering the composition containing the ADP-ribose binding peptide or a pharmaceutically acceptable salt thereof as an active ingredient. In addition, the term "prevention" refers to any activity that inhibits or delays the onset of cancer by administering the composition.

The weight percent of the ADP-ribose binding peptide or a pharmaceutically acceptable salt thereof contained in the pharmaceutical composition is not particularly limited, but may be 0.0001 to 90 wt%, specifically 0.001 to 50 wt%, and more specifically, 0.01 to 20 wt%, based on the total weight of the final composition.

The pharmaceutical composition may be prepared by further containing appropriate carriers, excipients or diluents commonly used in the manufacture of pharmaceuticals. Specifically, each of the pharmaceutical compositions of the present disclosure may be formulated according to conventional methods in the form of powders, granules, tablets, capsules, suspensions, emulsions, syrups, aerosols, oral formulations such as oral patches, and the like, external preparations, external patches, suppositories, and sterile injections.

When the pharmaceutical composition is used for oral administration, it may be prepared as a sustained-release preparation through appropriate encapsulation, enteric coating, polymer blend, and the like.

In an embodiment, the sustained-release preparation may be prepared as a long-acting preparation.

In an embodiment, the long-acting preparation may be mixed with a polymer and a lipid in an appropriate ratio.

The pharmaceutical composition of the present disclosure may be administered to a subject who has developed or is at risk of developing cancer. As used herein, the term "subject" means all animals including humans.

The pharmaceutical composition of the present disclosure may be administered to a subject in a pharmaceutically effective amount. As used herein, the term "administration" refers to introducing the pharmaceutical composition of the present disclosure to a target subject by any suitable method, and the administration may be performed through various oral or parenteral routes as long as it is possible to reach the target tissue. Examples of the administration route may include, but are not limited to, oral, intramuscular, intravenous, arterial, subcutaneous, intraperitoneal, pulmonary, and nasal routes, and for example, may include subcutaneous or oral administration.

As used herein, the term "pharmaceutically effective amount" means an amount sufficient to prevent and/or treat cancer with a reasonable benefit/risk ratio applicable to medical use. Appropriate dosage and frequency of administration may be selected according to a method known in the art, wherein the dosage and frequency of administration of the pharmaceutical composition of the present disclosure to be actually administered may be appropriately determined by various factors such as the type of symptom to be treated, route of administration, gender, health condition, diet, age and weight of the subject, and severity of the disease.

In the present disclosure, the pharmaceutically acceptable salt refers to salts commonly used in the pharmaceutical industry, and may include, for example, salts of inorganic ions including sodium, potassium, calcium, magnesium, lithium, copper, manganese, zinc, iron, and the like, and salts of inorganic acids such as hydrochloric acid, phosphoric acid, and sulfuric acid, and in addition thereto, may include salts of organic acids such as ascorbic acid, citric acid, tartaric acid, lactic acid, maleic acid, malonic acid, fumaric acid, glycolic acid, succinic acid, propionic acid, acetic acid, orotate acid, acetylsalicylic acid, and the like, and amino acid salts such as lysine, arginine, guanidine, and the like. Further, organic ion salts such as tetramethyl ammonium, tetraethyl ammonium, tetrapropyl ammonium, tetrabutyl ammonium, benzyl trimethyl ammonium, and benzethonium capable of being used in pharmaceutical reactions, purification, and separation processes, may be included. However, the types of salts meant in the present disclosure are not limited by these listed salts.

Since the ADP-ribose binding peptide of the present disclosure has excellent anti-cancer activity, the ADP-ribose binding peptide may be prepared in the form of a functional food composition as well as a pharmaceutical composition.

When the composition of the present disclosure is prepared in the form of a food composition, the food composition may include additional ingredients that are commonly used in food and are able to improve odor, taste, and visual properties. For example, food additives may be added. The additive is selected according to the type of food and used in an appropriate amount.

The food composition may be prepared as a health functional food, wherein the health functional food is the same term as food for special health use (FoSHU), which refers to food with high medical and medical effects processed to efficiently show bioregulatory functions in addition to nutritional supply. The health functional food may be prepared in various forms such as tablets, capsules, powders, granules, liquids, and pills in order to obtain useful effects for the improvement of cancer.

Still another aspect of the present disclosure is a pharmaceutical composition for an anti-cancer adjuvant to enhance reactivity to a second anti-cancer drug, comprising: the ADP-ribose binding peptide as described above or a pharmaceutically acceptable salt thereof as an active ingredient.

Still another aspect of the present disclosure is a pharmaceutical composition for preventing or treating cancer, comprising: (i) the ADP-ribose binding peptide as described above or a pharmaceutically acceptable salt thereof, and (ii) a second anti-cancer drug as an active ingredient.

The ADP-ribose binding peptide and a pharmaceutically acceptable salt thereof are the same as described above.

The ADP-ribose binding peptide of the present disclosure also has a very excellent effect as a pharmaceutical composition for anti-cancer adjuvant for the purpose of enhancing the reactivity to the second anti-cancer drug in addition to having an anti-cancer effect alone. Therefore, it is possible to use a pharmaceutical composition for preventing or treating cancer, comprising: the second anti-cancer drug as an active ingredient together with the ADP-ribose binding peptide or a pharmaceutically acceptable salt thereof.

As used herein, the term "second anti-cancer drug" refers to any drug having anti-cancer activity other than the ADP-ribose binding peptide of the present disclosure. In the present disclosure, the scope of the second anti-cancer drug is not particularly limited, and those skilled in the art may select and use an appropriate type of the second anti-cancer drug according to the purpose of cancer cure, control, symptom relief, and the like, and depending on the type and stage of cancer. The second anti-cancer drug may be, for example, a cytotoxic anti-cancer drug, a targeted anti-cancer drug, an immune anti-cancer drug, or a metabolic anti-cancer drug, but is not limited thereto.

In the present disclosure, the cytotoxic anti-cancer drug is a drug that exhibits an anti-cancer effect by attacking cancer cells that divide indiscriminately at a faster rate than normal cells, which means the same as commonly used in the technical field to which the present disclosure belongs. The cytotoxic anti-cancer drug includes alkylating agents, antimetabolites, and natural anti-cancer drugs.

The alkylating agent may include nitrogen mustards (for example, cyclophosphamide, chlormethine, uramustine, melphalan, chlorambucil, ifosfamide, bendamustine, and the like), alkyl sulfonates (for example, busulfan, procarbazine, and the like), nitrosoureas (for example, carmustine, lomustine, streptozocin, and the like), platinum-based alkylating agents (for example, cisplatin, carboplatin, dicycloplatin, eptaplatin, lobaplatin, myriplatin, nedaplatin, oxaliplatin, picoplatin, satraplatin, triplatin tetranitrate, and the like), but is not limited thereto. The alkylating agent may cause destruction of cancer cells by binding to DNA in cancer cells and damaging the DNA structure.

The antimetabolite may include pyrimidine derivatives (for example, 5-fluorouracil, capecitabine, cystarabine, gemcitabine, fludarabine, and the like), folate derivatives (for example, methotrexate, pemetrexed, and the like), purine derivatives (for example, mercaptopurine, and the like), but is not limited thereto. The antimetabolite may induce cancer cell death by inhibiting metabolism necessary for cell survival and DNA replication.

The natural anti-cancer drug may include topoisomerase inhibitors (for example, camptothecin, epipodophyllotoxin, taxane-based drugs (docetaxel, paclitaxel)), antibiotics (for example, dactinomycin, doxorubicin, daunorubicin, mitomycin, phleomycin, idarubicin, mitoxantrone HCl, and the like), but is not limited thereto.

In the present disclosure, the targeted anti-cancer drug is an anti-cancer drug that induces the death of cancer cells by inhibiting a target protein (receptor or enzyme) involved in cancer growth, which means the same as commonly used in the technical field to which the present disclosure belongs. The cytotoxic anti-cancer drug includes target protein (such as tyrosine kinase, etc.) inhibitory small molecule compounds and monoclonal antibodies.

For example, the targeted anti-cancer drug may be a receptor tyrosine kinase inhibitor targeting at least one target selected from the group consisting of VEGF-A and EGFR.

In an embodiment, the targeted anti-cancer drug capable of being administered in combination with the ADP-ribose binding peptide is a VEGF-A inhibitor. In the present disclosure, the VEGF-A inhibitor may include monoclonal antibodies such as bevacizumab, ranibizumab, aflibercept, and ramucirumab, and small molecule compounds such as sunitinib, pazopanib, sorafenib, and axitinib, but is not limited thereto.

In an embodiment, the targeted anti-cancer drug capable of being administered in combination with the ADP-ribose binding peptide is an EGFR inhibitor. In the present disclosure, the EGFR inhibitor may include monoclonal antibodies such as cetuximab, panitumumab, zalutumumab, nimotuzumab, and matuzumab together with small molecule compounds such as osimertinib, gefitinib, erlotinib, afatinib, brigatinib, icotinib, and vandetanib, but is not limited thereto.

In addition, the targeted anti-cancer drug of the present disclosure may also include HER2-targeted anti-cancer drugs such as lapatinib, neratinib, and afatinib; Bcr-Abl targeted anti-cancer drugs such as imatinib, dasatinib, and nilotinib; Src targeted anti-cancer drugs such as bosutinib; JAK targeted anti-cancer drugs such as lestaurtinib, ruxolitinib, and pacritinib; MAP2 Kinase targeted anti-cancer drugs such as cobimethinib, selumetinib, trametinib, and binimetinib; MEL4-ALK targeted anti-cancer drugs such as ceritibin and crizotinib, but is not particularly limited in view of the kind.

In addition, the second anti-cancer drug of the present disclosure may be a combination of one or more cytotoxic anti-cancer drugs and/or targeted anti-cancer drugs, which may be administered at the same time or at different times.

In the present disclosure, an immuno-oncology drug refers to drugs that activate the body's immune system to fight cancer cells. In the present disclosure, the immuno-oncology drug may include immune checkpoint inhibitors, immune cell therapeutic agents, anti-cancer vaccines, and antibody-drug conjugates, wherein the appropriate type thereof may be selected to cure, control, and alleviate symptoms of cancer depending on the type and stage of cancer.

In an embodiment, the immuno-oncology drug may be an immune checkpoint inhibitor, and may be one or more selected from the group consisting of a PD-1 antibody, a PD-L1 antibody, a CTLA-4 antibody, a CD28 antibody, a KIR antibody, a TCR antibody, a LAG-3 antibody, a TIM-3 antibody, a TIGIT antibody, an A2aR antibody, an ICOS antibody, an OX40 antibody, a 4-1BB antibody, and a GITR antibody. For example, the immune checkpoint inhibitor may be PD-1 antibodies such as nivolumab, pembrolizumab, cemiplimab, pidilizumab, toripalimab; PD-L1 antibodies such as atezolizumab, avelumab, duralumab; CTLA-4 antibodies such as ipilimumab and tremelimumab, but is not limited thereto.

In an embodiment, the immuno-oncology drug may be an immune cell therapeutic agent, and may be a chimeric antigen receptor-natural killer (CAR)-NK agent or a chimeric antigen receptor (CAR)-T agents such as tisagenlecleucel or axicabtagene ciloleucel, but is not limited thereto.

In the present disclosure, a cancer metabolism drug refers to a drug that is involved in the growth and survival of cancer cells, such as supplying nutrients to cancer cells, or is involved in various essential metabolic reactions to kill cancer cells. The cancer metabolism drug may include, for example, IM-156, 3-bromopyruvic acid (3BP), NYH817100, WZB117, GNE-140, AZ93, AZD3965, CPI-613, MKT-077, CB-839, CB-1158, CPI-444, TVB-2640, NDI-010976, TCD-717, ADI-PEG20, Epacadostat, Indoximod, PX478, CPI-0610, RTA402, APO866, GMX1778, AG-221 or AG-120, and the like, but is not limited thereto.

Since the ADP-ribose binding peptide of the present disclosure has an effect of enhancing reactivity to the second anti-cancer drug by disrupting the balance of ADP-ribose in cancer cells, thus being usable as an anti-cancer adjuvant, the type of second anti-cancer drug used or the type of cancer is not particularly limited. For an example, cancer may be solid tumors such as brain cancer, lung cancer, pancreatic cancer, liver cancer, breast cancer, colon cancer, kidney cancer, gastric cancer, or ovarian cancer, but is not limited thereto.

The ADP-ribose binding peptide of the present disclosure may be administered in combination with the second anti-cancer drug in a form that exists independently of each other, or depending on the purpose, may be administered in a state in which a physical/chemical bond is formed with the second anti-cancer drug by any method known in the art. For example, the ADP-ribose binding peptide may be used in a state of being directly coupled to the second anti-cancer drug, or may be used in a state of being connected to the second anti-cancer drug through a known linker. An application method of the ADP-ribose binding peptide of the present disclosure is not particularly limited as long as the ADP-ribose binding peptide acts together with the second anti-cancer drug to exhibit a synergistic anti-cancer effect.

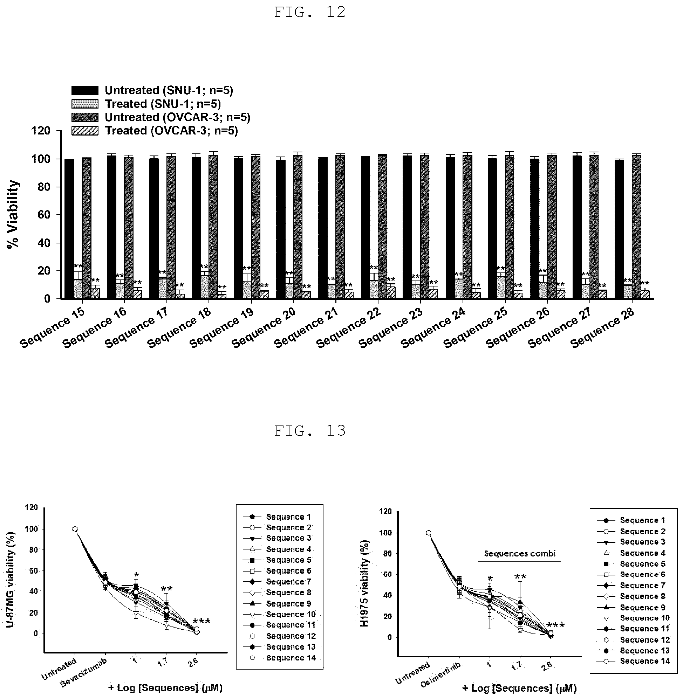

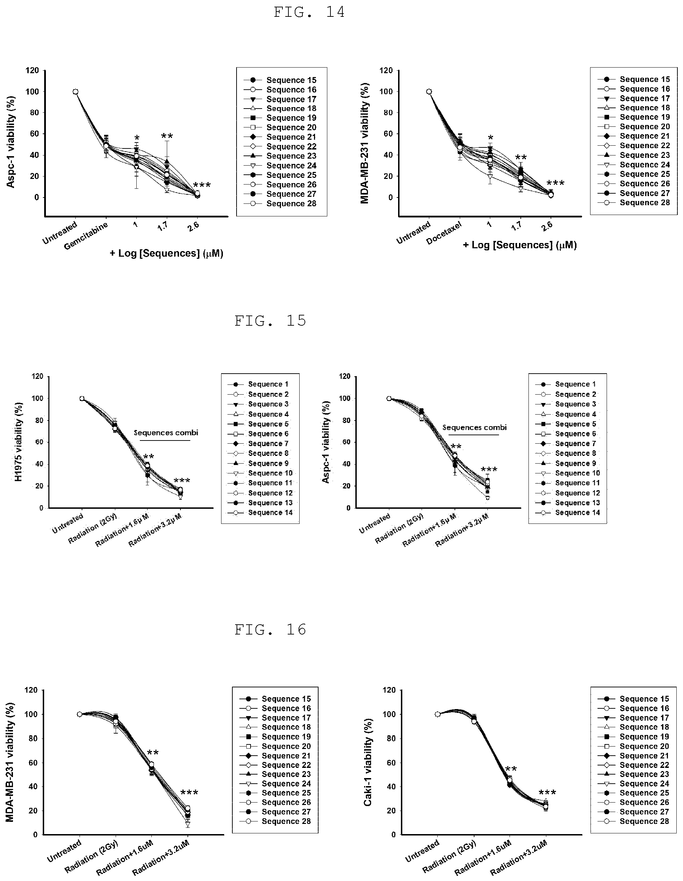

In a specific embodiment of the present disclosure, it was confirmed that when different cancer types were treated with the ADP-ribose binding peptide of the present disclosure together with low concentrations of bevacizumab, osimertinib, gemcitabine, and docetaxel, the reactivity to the anti-cancer drug was enhanced in all cancer cells (FIGS. 13 and 14). Therefore, the ADP-ribose binding peptide of the present disclosure may enhance the reactivity of the second anti-cancer drug, which is capable of being employed very usefully as an anti-cancer adjuvant, and may have excellent anti-cancer activity even when the second anti-cancer drug is treated at a low concentration, thereby making it possible to minimize side effects that may be caused by the second anti-cancer drug.

Still another aspect of the present disclosure is a pharmaceutical composition for an anti-cancer adjuvant to enhance reactivity to anti-cancer radiation therapy, comprising: the ADP-ribose binding peptide as described above or a pharmaceutically acceptable salt thereof as an active ingredient.

The ADP-ribose binding peptide and a pharmaceutically acceptable salt thereof are the same as described above.

As used herein, the term "anti-cancer radiation therapy" refers to a therapeutic action of irradiating cancer cells or tumor tissues with radiation for the purpose of killing cancer cells. In general, anti-cancer radiation therapy is a standard treatment for controlling unresectable or inoperable tumors, or tumor metastasis, and is based on the principle that radiation delivered to a target site causes the death of reproductive cells. The anti-cancer radiation therapy in the present disclosure may be ionizing radiation therapy, electromagnetic radiation therapy, brachytherapy or external beam radiation therapy, but is not limited thereto.

Since the composition containing the ADP-ribose binding peptide or a pharmaceutically acceptable salt thereof according to the present disclosure exhibits a synergistic anti-cancer effect when used in combination with radiation therapy, the composition may be usefully utilized as an anti-cancer adjuvant for radiation therapy or a radiation sensitizer that improves radiation sensitivity, wherein the type of applicable cancer is not particularly limited. As an example, cancer may be solid tumors such as brain cancer, lung cancer, pancreatic cancer, liver cancer, breast cancer, colon cancer, kidney cancer, gastric cancer, and ovarian cancer, but is not limited thereto.

As an embodiment, the solid tumor may be resistant to radiation therapy. Since the anti-cancer effect of radiation therapy is exhibited by the formation of DNA cleavage, resistance to radiation therapy is generally determined by a mechanism capable of repairing DNA damage induced by radiation therapy. As a method of increasing the anti-cancer efficacy of radiation therapy, a number of attempts have been made to inhibit DNA repair mechanisms, and the sensitivity of radiation therapy may be increased when the repair of broken DNA strands is blocked. The two main types of DNA damage are single-strand breaks (SSB) and double-strand breaks (DSB). Therefore, the categories of two repair pathways targeting SSB and DSB may be described as examples. Base Excision Repair (BER) is one of several pathways involved in the repair of selected types of DNA SSBs.

PARP1 plays an important role in the BER of DNA SSBs through a process known as ADP-ribosylation. In the nucleus, PARP1 detects damage to SSB DNA, and for damage repair, recruits DNA repair complexes via ADP-ribosylation to SSB sites. Since over-accumulation of poly-ADP-ribose synthesized through ADP-ribosylation by PARP-1 activity ultimately leads to cell death, in order to prevent this phenomenon, cancer cells activate survival signaling activities by activating the degradation of poly-ADP-ribose through proteasomes such as PARG, ARH3, and the like. The present inventors noticed that the accumulation of ADP-ribose or ADP-ribose polymer in cancer cells could act as a vehicle capable of disrupting these biochemical survival mechanisms of cancer cells, thereby making it possible to be used as an important anti-cancer adjuvant to overcome resistance to radiation therapy.

In a specific embodiment of the present disclosure, it was confirmed that when the ADP-ribose binding peptide of the present disclosure was treated in combination with lowdose radiation in different cancer types, the reactivity to radiation therapy was enhanced in all cancer types (FIGS. 15 and 16). Therefore, the ADP-ribose binding peptide of the present disclosure is able to enhance the reactivity to radiation therapy to thereby be very useful as an anti-cancer adjuvant, and even when irradiated with radiation at a tolerable dose, excellent anti-cancer activity may be obtained, which may minimize side effects that may occur due to radiation.

The embodiments of the present disclosure are provided to more completely explain the present disclosure to those skilled in the art. Further, "comprising" a component throughout the specification does not mean excluding other components, but rather it means that other components may be further included, unless otherwise stated.

### [Mode for Carrying out the Invention]

Hereinafter, the constitution and effects of the present disclosure will be described in more detail through Examples. These Examples are only provided for illustrating the present disclosure, but the scope of the present disclosure is not limited by these Examples, but by the claims.

### Examples 1 to 14. Preparation of adenosine diphosphate (ADP)-ribose binding peptides

The ADP-ribose binding peptides of Examples 1 to 14 were synthesized from WWE domains present in various types of proteins, and were used in experiments. The specific information on the peptides used is shown in Table 1 below.

**[Table 1]**

| **Example** | **Sequence information (SEQ ID NO)** | **Derived protein** |
|---|---|---|
| 1 | VPYIIDLQSMHQFRQDTGTMRPVRR (SEQ ID NO. 1) | DTX1 |
| 2 | APYIIDLQSMNQFRQDTGTLRPVRR (SEQ ID NO. 2) | DTX4 |
| 3 | APYIIDLPSWTQFRQDTGTMRAVRR (SEQ ID NO. 3) | DTX2 |
| 4 | RVYTIDFNSMQQINEDTGTARAIQR (SEQ ID NO. 4) | TRIP12 |
| 5 | FCYLIYFNSMSQMNRQTRRRRRLRR (SEQ ID NO. 5) | PARP7 |
| 6 | FSYVIDFNTMGQINRQTQRQRRVRR (SEQ ID NO. 6) | PARP12 |
| 7 | FSYKIDFAEMKQMNLTTGKQRLIKR (SEQ ID NO. 7) | PARP11 |
| 8 | YNYTVNYTTHTQTNKTSSFCRSVRR (SEQ ID NO. 8) | PIK3C2G |
| 9 | RRYTVQFTTMVQVNEETGNRRPVM (SEQ ID NO. 9) | HUWE1 |
| 10 | WIWYWKNESGTWIQYGEEKDKRKN (SEQ ID NO. 10) | ZC3HAV1 |
| 11 | FLYVADLENMVQYRRNEHGRRRKIKR (SEQ ID NO. 11) | RNF146 |
| 12 | RHYTVNLNTYTATDTKGHSLSVQR (SEQ ID NO. 12) | PARP14 |
| 13 | GRYDVHLGERMRYAVYWDELASEVRR (SEQ ID NO. 13) | DDHD2 |
| 14 | TAYEASVCDYLEQQVARGN (SEQ ID NO. 14) | HNRNPA2B1 |

In addition, peptides of Examples 15 to 28 were prepared by binding a cell-penetrating peptide to the N-terminus of the peptides of Examples 1 to 14. Peptide sequences of Examples 15 to 28 are shown in Table 2.

**[Table 2]**

| **Example** | **Sequence information (SEQ ID NO)** |
|---|---|
| 15 | GRKKRRQRRRPQVPYIIDLQSMHQFRQDTGTMRPVRR (SEQ ID NO. 15) |
| 16 | GRKKRRQRRRPQAPYIIDLQSMNQFRQDTGTLRPVRR (SEQ ID NO. 16) |
| 17 | GRKKRRQRRRPQAPYIIDLPSWTQFRQDTGTMRAVRR (SEQ ID NO. 17) |
| 18 | GRKKRRQRRRPQRVYTIDFNSMQQINEDTGTARAIQR (SEQ ID NO. 18) |
| 19 | GRKKRRQRRRPQFCYLIYFNSMSQMNRQTRRRRRLRR (SEQ ID NO. 19) |
| 20 | GRKKRRQRRRPQFSYVIDFNTMGQINRQTQRQRRVRR (SEQ ID NO. 20) |
| 21 | GRKKRRQRRRPQFSYKIDFAEMKQMNLTTGKQRLIKR (SEQ ID NO. 21) |
| 22 | GRKKRRQRRRPQYNYTVNYTTHTQTNKTSSFCRSVRR (SEQ ID NO. 22) |
| 23 | GRKKRRQRRRPQRRYTVQFTTMVQVNEETGNRRPVM (SEQ ID NO. 23) |
| 24 | GRKKRRQRRRPQWIWYWKNESGTWIQYGEEKDKRKN (SEQ ID NO. 24) |
| 25 | GRKKRRQRRRPQFLYVADLENMVQYRRNEHGRRRKIKR (SEQ ID NO. 25) |
| 26 | GRKKRRQRRRPQRHYTVNLNTYTATDTKGHSLSVQR (SEQ ID NO. 26) |
| 27 | GRKKRRQRRRPQGRYDVHLGERMRYAVYWDELASEVRR (SEQ ID NO. 27) |
| 28 | GRKKRRQRRRPQTAYEASVCDYLEQQVARGN (SEQ ID NO. 28) |

### Experimental Example 1: Confirmation of change in adenosine diphosphate (ADP)-ribose level in cancer cells

The peptides of Examples 1 to 28 of the present disclosure are designed to inhibit the action of poly ADP-ribose degradation enzyme. In Experimental Example 1, it was confirmed through cell experiments whether poly ADP-ribose was inhibited from being degraded by each of the peptides and accumulated in cancer cells.

### 1-1. Change in ADP-ribose level in U-87MG cell

5 × 10⁵ U-87MG cells were divided into a group not treated with the peptide of Example and a group treated with 0.2, 1, 2, 4, 8, 16, and 32 µM of the peptide of Example 1 (SEQ ID NO: 1) or Example 8 (SEQ ID NO: 8), and cultured for 24 hours under conditions of 37 °C and 5 % CO₂ in Eagle's minimum essential medium (EMEM) supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

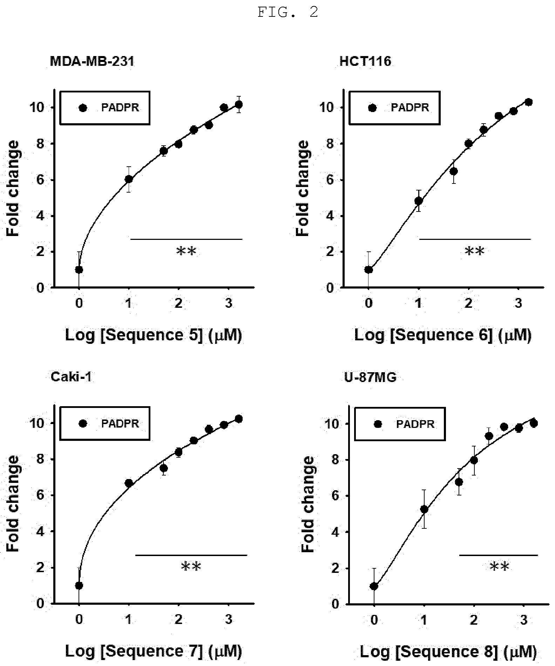

The top left of FIG. 1 and the bottom right of FIG. 2 showed the amount of increase in ADP-ribose (expressed as fold change) in U-87MG cells compared to the control group according to the peptide treatment of Example 1 or 8, indicating that compared to the untreated group, both groups of the treated peptides showed a significant increase in the amount of ADP-ribose in a concentration-dependent manner (** p < 0.001).

### 1-2. Change in ADP-ribose level in H1975 cell

5 × 10⁵ H1975 cells were divided into a group not treated with the peptide of Example and a group treated with 0.2, 1, 2, 4, 8, 16, and 32 µM of the peptide of Example 2 (SEQ ID NO: 2), and cultured for 24 hours under conditions of 37 °C and 5 % CO₂ in RPMI-1640 medium supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

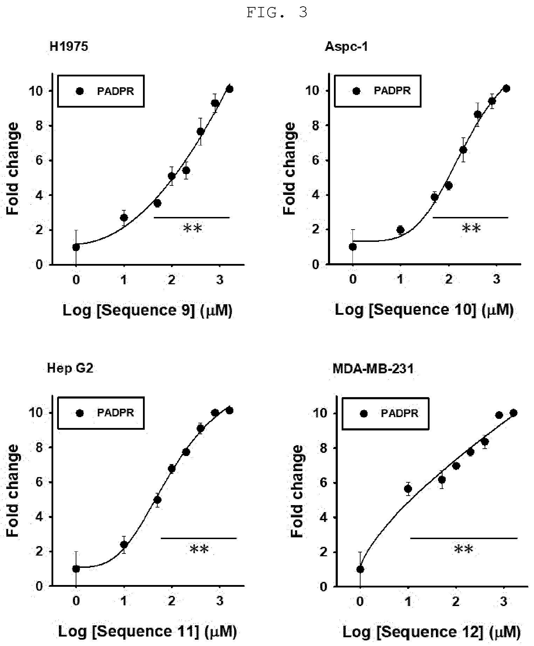

The top right of FIG. 1 and the top left of FIG. 3 showed the amount of increase in ADP-ribose (expressed as fold change) in H1975 cells compared to the control group according to the peptide treatment of Example 2 or 9, indicating that compared to the untreated group, the treated peptide group showed a significant increase in the amount of ADP-ribose in a concentration-dependent manner (** p < 0.001).

### 1-3. Change in ADP-ribose level in Aspc-1 cell

5 × 10⁵ Aspc-1 cells were divided into a group not treated with the peptide of Example and a group treated with 0.2, 1, 2, 4, 8, 16, and 32 µM of the peptide of Example 3 (SEQ ID NO: 3), and cultured for 24 hours under conditions of 37 °C and 5 % CO₂ in RPMI-1640 medium supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

The bottom left of FIG. 1 and the top right of FIG. 3 showed the amount of increase in ADP-ribose (expressed as fold change) in Aspc-1 cells compared to the control group according to the peptide treatment of Example 3 or 10, indicating that compared to the untreated group, the treated peptide group showed a significant increase in the amount of ADP-ribose in a concentration-dependent manner (** p < 0.001).

### 1-4. Change in ADP-ribose level in Hep G2 cell

5 × 10⁵ Hep G2 cells were divided into a group not treated with the peptide of Example and a group treated with 0.2, 1, 2, 4, 8, 16, and 32 µM of the peptide of Example 4 (SEQ ID NO: 4), and cultured for 24 hours under conditions of 37 °C and 5 % CO₂ in EMEM medium supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

The bottom right of FIG. 1 and the bottom left of FIG. 3 showed the amount of increase in ADP-ribose (expressed as fold change) in Hep G2 cells compared to the control group according to the peptide treatment of Example 4 or 11, indicating that compared to the untreated group, the treated peptide group showed a significant increase in the amount of ADP-ribose in a concentration-dependent manner (** p < 0.001).

### 1-5. Change in ADP-ribose level in MDA-MB-231 cell

5 × 10⁵ MDA-MB-231 cells were divided into a group not treated with the Example peptide and a group treated with 0.2, 1, 2, 4, 8, 16, and 32 µM of the peptide of Example 5 (SEQ ID NO: 5), and cultured for 24 hours under conditions of 37 °C and 5 % CO₂ in Leibovitz's L-15 medium supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

The top left of FIG. 2 and the bottom right of FIG. 3 showed the amount of increase in ADP-ribose (expressed as fold change) in MDA-MB-231 cells compared to the control group according to the peptide treatment of Example 5 or 12, indicating that compared to the untreated group, the treated peptide group showed a significant increase in the amount of ADP-ribose in a concentration-dependent manner (** p < 0.001).

### 1-6. Change in ADP-ribose level in HCT116 cell

5 × 10⁵ HCT116 cells were divided into a group not treated with the peptide of Example and a group treated with 0.2, 1, 2, 4, 8, 16, and 32 µM of the peptide of Example 6 (SEQ ID NO: 6), and cultured for 24 hours under conditions of 37 °C and 5 % CO₂ in McCoy's 5A medium supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

The top right of FIG. 2 and the left graph of FIG. 4 show the amount of increase in ADP-ribose as fold change in HCT116 cells compared to the control group according to the peptide treatment of Example 6 or 13, indicating that compared to the untreated group, the treated peptide group showed a significant increase in the amount of ADP-ribose in a concentration-dependent manner (** p < 0.001).

### 1-7. Change in ADP-ribose level in Caki-1 cell

5 × 10⁵ Caki-1 cells were divided into a group not treated with the peptide of Example and a group treated with 0.2, 1, 2, 4, 8, 16, and 32 µM of the peptide of Example 7 (SEQ ID NO: 7), and cultured for 24 hours under conditions of 37 °C and 5 % CO₂ in McCoy's 5A medium supplemented with 10 % FBS, 100 units/ml penicillin, and 100 µg/ml streptomycin. The medium of the cultured cells was removed, and the samples were treated with RIPA buffer and 1 % SDS. The prepared supernatant was used for ELISA analysis to detect and analyze ADP-ribose.

The bottom left of FIG. 2 and the right graph of FIG. 4 shows the amount of increase in ADP-ribose as fold change in Caki-1 cells compared to the control group according to the peptide treatment of Example 7 or 14, indicating that compared to the untreated group, the treated peptide group showed a significant increase in the amount of ADP-ribose in a concentration-dependent manner (** p < 0.001).

### Experimental Example 2: Change in cancer cell viability according to peptide treatment of Examples

Cells maintain a balance between the production and degradation of ADP-ribose while maintaining biochemical homeostasis. Meanwhile, since cancer cells continuously divide and grow rapidly, if this balance is disturbed, the survival of cancer cells may be significantly affected compared to normal cells.

Referring to the results of Experimental Example 1 above, when cancer cells are treated with the peptides of Examples of the present disclosure, the amount of ADP-ribose in the cells is significantly increased, and thus the homeostatic balance of cancer cells may be disturbed. Thus, in Experimental Example 2, the viability of cancer cells according to the treatment with the peptides of Examples 1 to 28 was measured.

### 2-1. Change in viability in U-87MG cells according to treatment with ADP-ribose binding peptide

After culturing 3 × 10³ U-87MG cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl) -2, 5-diphenyl tetrazolium bromide reagent was added to each well, followed by reaction for 1 hour. After the reaction was completed, the reagent was removed, 200 µl of dimethyl sulfoxide was added to each well, and the absorbance was measured to confirm cell viability.

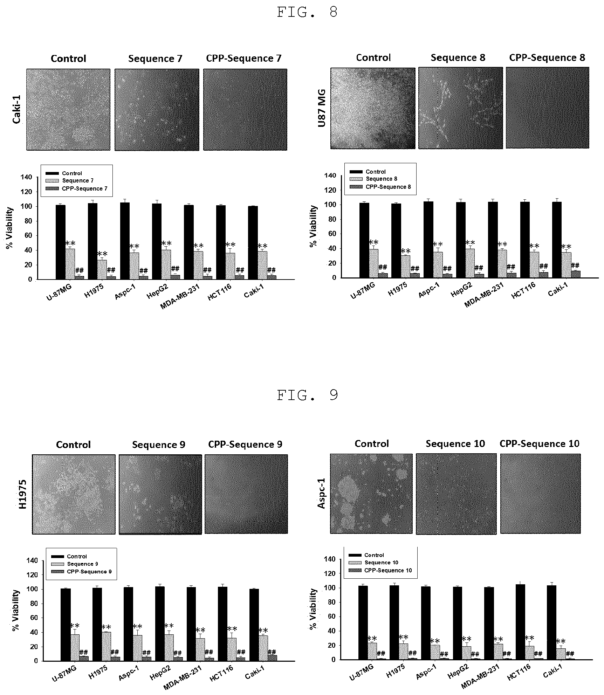

The top left of FIG. 5 and the top right of FIG. 8 are representative photomicrographs of U-87MG cell groups treated with and without the peptides of Examples 1, 15, 8, and 22, respectively. In common, it could be observed that the group not treated with the peptide showed rapid growth of U-87MG cells, whereas the groups treated with the peptides of Examples of the present disclosure showed that U-87MG cells were inhibited from growing and killed. In particular, it was confirmed that cancer cells were completely killed in the groups treated with the cell-penetrating peptide (CPP)-attached peptides of Examples 15 and 22.

### 2-2. Change in viability in H1975 cells according to treatment with ADP-ribose binding peptide

After culturing 3 × 10³ H1975 cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl) -2, 5-diphenyl tetrazolium bromide reagent was added to each well, followed by reaction for 1 hour. After the reaction was completed, the reagent was removed, 200 µl of dimethyl sulfoxide was added to each well, and the absorbance was measured to confirm cell viability.

The top right of FIG. 5 and the top left of FIG. 9 are representative photomicrographs of H1975 cell groups treated with and without the peptides of Examples 2, 16, 9, and 23, respectively. In common, it could be observed that the group not treated with the peptide showed a rapid growth of H1975 cells, whereas the groups treated with the peptides of Examples of the present disclosure showed that H1975 cells were inhibited from growing and killed. In particular, it was confirmed that cancer cells were completely killed in the groups treated with the CPP-attached peptides of Examples 16 and 23.

### 2-3. Change in viability in Aspc-1 cells according to treatment with ADP-ribose binding peptide

After culturing 3 × 10³ Aspc-1 cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl)-2, 5-diphenyl tetrazolium bromide reagent was added to each well, followed by reaction for 1 hour. After the reaction was completed, the reagent was removed, 200 µl of dimethyl sulfoxide was added to each well, and the absorbance was measured to confirm cell viability.

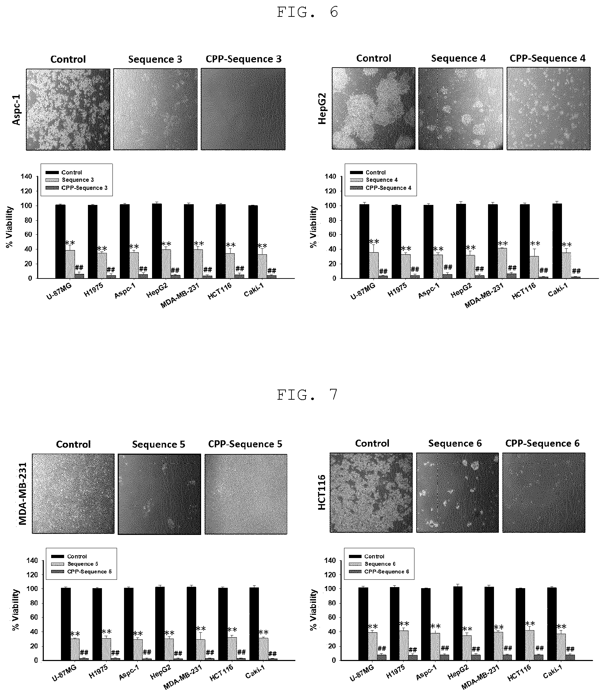

The top left of FIG. 6 and the top right of FIG. 9 are representative photomicrographs of Aspc-1 cell groups treated with and without the peptides of Examples 3, 17, 10, and 24, respectively. In common, it could be observed that the group not treated with the peptide showed rapid growth of Aspc-1 cells, whereas the groups treated with the peptides of Examples of the present disclosure showed that Aspc-1 cells were inhibited from growing and killed. In particular, it was confirmed that cancer cells were completely killed in the groups treated with the CPP-attached peptides of Examples 17 and 24.

### 2-4. Change in viability in Hep G2 cells according to treatment with ADP-ribose binding peptide

After culturing 3 × 10³ Hep G2 cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl)-2, 5-diphenyl tetrazolium bromide reagent was added to each well, followed by reaction for 1 hour. After the reaction was completed, the reagent was removed, 200 µl of dimethyl sulfoxide was added to each well, and the absorbance was measured to confirm cell viability.

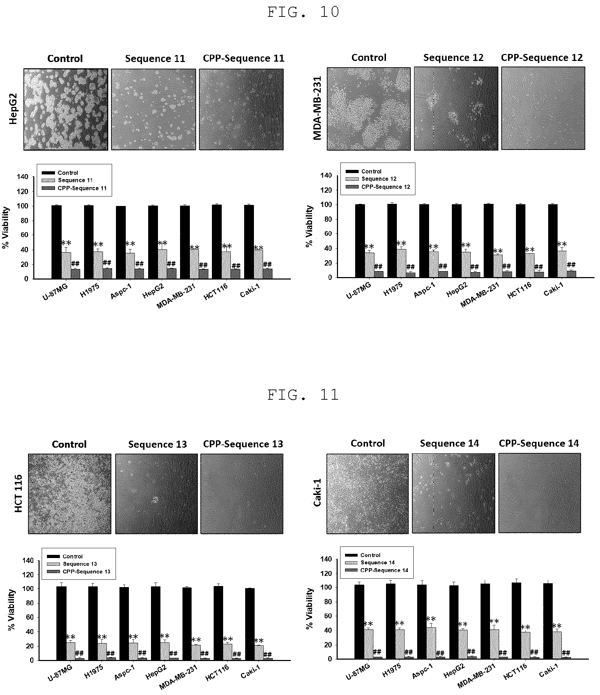

The top right of FIG. 6 and the top left of FIG. 10 are representative photomicrographs of Hep G2 cell groups treated with and without the peptides of Examples 4, 18, 11, and 25, respectively. In common, it could be observed that the group not treated with the peptide showed a rapid growth of Hep G2 cells, whereas the groups treated with the peptides of Examples of the present disclosure showed that Hep G2 cells were inhibited from growing and killed. In particular, it was confirmed that cancer cells were completely killed in the groups treated with the CPP-attached peptides of Examples 18 and 25.

### 2-5. Change in viability in MDA-MB-231 cells according to treatment with ADP-ribose binding peptide

After culturing 3 × 10³ MDA-MB-231 cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl)-2, 5-diphenyl tetrazolium bromide reagent was added to each well, followed by reaction for 1 hour. After the reaction was completed, the reagent was removed, 200 µl of dimethyl sulfoxide was added to each well, and the absorbance was measured to confirm cell viability.

The top left of FIG. 7 and the top right of FIG. 10 are representative photomicrographs of MDA-MB-231 cell groups treated with and without the peptides of Examples 5, 19, 12, and 26, respectively. In common, it could be observed that the group not treated with the peptide showed rapid growth of MDA-MB-231 cells, whereas the groups treated with the peptides of Examples of the present disclosure showed that MDA-MB-231 cells were inhibited from growing and killed. In particular, it was confirmed that cancer cells were completely killed in the groups treated with the CPP-attached peptides of Examples 19 and 26.

### 2-6. Change in viability in HCT116 cells according to treatment with ADP-ribose binding peptide

After culturing 3 × 10³ HCT116 cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl) -2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

The top right of FIG. 7 and the top left of FIG. 11 are representative photomicrographs of HCT116 cell groups treated with and without the peptides of Examples 6, 20, 13, and 27, respectively. In common, it could be observed that the group not treated with the peptide showed rapid growth of HCT116 cells, whereas the groups treated with the peptides of Examples of the present disclosure showed that HCT116 cells were inhibited from growing and killed. In particular, it was confirmed that cancer cells were completely killed in the groups treated with the CPP-attached peptides of Examples 20 and 27.

### 2-7. Change in viability in Caki-1 cells according to treatment with ADP-ribose binding peptide

After culturing 3 × 10³ Caki-1 cells in a 96-well plate for 24 hours under conditions of 37 °C and 5 % CO₂, the cells were either untreated or treated with the peptides of Examples 1 to 28 at a concentration of 16 µM, respectively. Then, the cells were cultured for 24 hours under conditions of 37 °C and 5 % CO₂, and 10 µl of 3-(4,5-dimethylthiazol-2-yl) -2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

The top left of FIG. 8 and the top right of FIG. 11 are representative photomicrographs of Caki-1 cell groups treated with and without the peptides of Examples 7, 21, 14, and 28, respectively. In common, it could be observed that the group not treated with the peptide showed rapid growth of Caki-1 cells, whereas the groups treated with the peptides of Examples of the present disclosure showed that Caki-1 cells were inhibited from growing and killed. In particular, it was confirmed that cancer cells were completely killed in the groups treated with the CPP-attached peptides of Examples 21 and 28.

### 2-8. Change in viability in SNU-1 cells and OVCAR-3 cells according to treatment with ADP-ribose binding peptide

After culturing 3 × 10³ SNU-1 cells or OVCAR-3 cells in a 96-well plate for 24 hours under conditions of 37 °C and 5 % CO₂, the cells were either untreated or treated with the peptides of Examples 15 to 28 at a concentration of 16 µM, respectively. Then, the cells were cultured for 96 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that most cancer cells were killed in the groups treated with the peptides of Examples 15 to 28, respectively, in both SNU-1 cells and OVCAR-3 cells (FIG. 12).

### Experimental Example 3: Change in cancer cell viability by combination treatment with peptides of Examples and low-concentration anti-cancer drugs

The anti-cancer effect, represented by the reduction of cancer cell survival, and the like, by existing anti-cancer drugs, may be enhanced by ADP-ribose signaling. Thus, it was expected that if the accumulation of ADP-ribose is induced while treating cancer cells with the existing anti-cancer drugs, a synergistic anti-cancer effect would be exhibited even if the cells are treated with existing anti-cancer drugs at a concentration below the required concentration. As confirmed in Experimental Example 1, the peptides of the present disclosure significantly increased the amount of ADP-ribose in cancer cells, and thus in Experimental Example 3, it was attempted to confirm whether a synergistic anti-cancer effect was exhibited at the time of combination treatment with peptides of Examples and a low-concentration anti-cancer drug.

### 3-1. Change in viability in U-87MG cells by combination treatment with peptides of Examples and low-concentration bevacizumab

After culturing 3 × 10³ U-87MG cells in a 96-well plate for 24 hours under conditions of 37 °C and 5 % CO₂, the cells were treated with 18 mM of bevacizumab alone or in combination with the peptides of Examples 1 to 14 at a concentration of 0.2, 1 or 8 µM. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that when U-87MG cells were treated with bevacizumab alone, about 49 % of all cancer cells were viable, but in all cases where bevacizumab and the peptides of Examples were treated in combination, the viability of cancer cells was significantly reduced even with a low concentration of bevacizumab (* p < 0.05, ** p < 0.001, *** p < 0.001; left image of FIG. 13).

### 3-2. Change in viability in H1975 cells by combination treatment of peptides of Examples and low-concentration osimertinib

After culturing 3 × 10³ H1975 cells in a 96-well plate for 24 hours under conditions of 37 °C and 5 % CO₂, the cells were treated with 1 nM osimertinib alone or in combination with the peptides of Examples 1 to 14 at a concentration of 0.2, 1 or 8 µM. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that when H1975 cells were treated with 1 nM osimertinib alone, about 49% of all cancer cells were viable, but in all cases where osimertinib and the peptides of Examples were treated in combination, the viability of cancer cells was significantly reduced even with a low concentration of osimertinib (* p < 0.05, ** p < 0.001, *** p < 0.001; right image of FIG. 13).

### 3-3. Change in viability in Aspc-1 cells by combination treatment of peptides of Examples and low-concentration gemcitabine

After culturing 3 × 10³ Aspc-1 cells in a 96-well plate for 24 hours under conditions of 37 °C and 5 % CO₂, the cells were treated with 1 nM gemcitabine_alone or in combination with the peptides of Examples 15 to 28 at a concentration of 0.2, 1 or 8 µM. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, when Aspc-1 cells were treated with gemcitabine alone, about 50% of all cancer cells survived, but in all cases where gemcitabine and the peptides of Examples were treated in combination, the viability of cancer cells was significantly reduced even with a low concentration of gemcitabine (* p < 0.05, ** p < 0.001, *** p < 0.001; left image of FIG. 14).

### 3-4. Change in viability in MDA-MB-231 cells by combination treatment of peptides of Examples and low-concentration docetaxel

After culturing 3 × 10³ MDA-MB-231 cells in a 96-well plate for 24 hours under conditions of 37 °C and 5 % CO2, the cells were treated with 50 µM docetaxel alone or in combination with the peptides of Examples 15 to 28 at a concentration of 0.2, 1 or 8 µM. Then, the cells were cultured for 24 hours under conditions of 37 °C and 5 % CO₂, 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, when MDA-MB-231 cells were treated with docetaxel alone, about 52 % of all cancer cells survived, but in all cases where docetaxel and the peptides of Examples were treated in combination, the viability of cancer cells was significantly reduced even with a low concentration of docetaxel (* p < 0.05, ** p < 0.001, *** p < 0.001; right image of FIG. 14).

### Experimental Example 4: Change in cancer cell viability by combination treatment with peptides of Examples and radiation

Radiation therapy may be expected to have an anti-cancer effect through the cell death action by damaging genetic material, but to overcome the anti-cancer effect, cancer cells continually repair DNA strands through a process known as ADP-ribosylation. However, it was expected that if ADP-ribose, which increases only temporarily due to the repair action, is supported continuously to accumulate by irradiation, there would be a synergistic anti-cancer effect even when irradiated with a tolerable dose of radiation. As confirmed in Experimental Example 1, the peptides of the Examples of the present disclosure significantly increased the amount of ADP-ribose in cancer cells, and thus in Experimental Example 4, the present inventors attempted to confirm whether a synergistic anti-cancer effect would be exhibited at the time of combination treatment of peptides of Examples and a tolerable dose of radiation.

### 4-1. Change in viability in H1975 cells by combination treatment of peptides of Examples and tolerable dose of radiation

After culturing 3 × 10³ H1975 cells in a 96-well plate for 24 hours under conditions of 37 °C and 5 % CO₂, the cells were treated with 2 Gy dose of radiation alone or in combination with 1.6 or 3.2 µM of the peptides of SEQ ID NOs: 1 to 14. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that when H1975 cells were treated with 2 Gy dose of radiation alone, about 76 % of cancer cells showed survival tolerance compared to the untreated group, but in all cases of combination treatment with the peptides of Examples, the viability of cancer cells was significantly reduced even with irradiation at a tolerable dose (** p < 0.001, *** p < 0.001; left image of FIG. 15).

### 4-2. Change in viability in Aspc-1 cells by combination treatment of peptides of Examples and tolerable dose of radiation

After culturing 3 × 10³ Aspc-1 cells in a 96-well plate for 24 hours under conditions of 37 °C and 5 % CO₂, the cells were treated with 2 Gy dose of radiation alone or in combination with 1.6 or 3.2 µM of the peptides of SEQ ID NOs: 1 to 14. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that when Aspc-1 cells were treated with 2 Gy dose of radiation alone, about 86 % of cancer cells showed survival tolerance compared to the untreated group, but in all cases of combination treatment with the peptides of Examples, the viability of cancer cells was significantly reduced even with irradiation at a tolerable dose (** p < 0.001, *** p < 0.001; right image of FIG. 15).

### 4-3. Change in viability in MDA-MB-231 cells by combination treatment of peptides of Examples and tolerable dose of radiation

After culturing 3 × 10³ MDA-MB-231 cells in a 96-well plate for 24 hours under conditions of 37°C and 5% CO₂, the cells were treated with 2 Gy dose of radiation alone or in combination with 1.6 or 3.2 µM of the peptides of SEQ ID NOs: 15 to 28. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that when MDA-MB-231 cells were treated with 2 Gy dose of radiation alone, about 86 % of cancer cells showed survival tolerance compared to the untreated group, but in all cases of combination treatment with the peptides of Examples, the viability of cancer cells was significantly reduced even with irradiation at a tolerable dose (** p < 0.001, *** p < 0.001; left image of FIG. 16).

### 4-4. Change in viability in Caki-1 cells by combination treatment of peptides of Examples and tolerable dose of radiation

After culturing 3 × 10³ Caki-1 cells in a 96-well plate for 24 hours under conditions of 37 °C and 5 % CO₂, the cells were treated with 2 Gy dose of radiation alone or in combination with 1.6 or 3.2 µM of the peptides of SEQ ID NOs: 15 to 28. Then, the cells were cultured for 24 hours under conditions of 37 °C and 5 % CO₂, and 10 µl of 3-(4,5-dimethylthiazol-2-yl)-2,5-diphenyltetrazolium bromide reagent was added to each well, followed by reaction for 1 hour. After the reaction was completed, the reagent was removed, 200 µl of dimethyl sulfoxide was added to each well, and the absorbance was measured to confirm cell viability.

As a result, it was confirmed that when Caki-1 cells were treated with 2 Gy dose of radiation alone, about 96 % of cancer cells showed survival tolerance compared to the untreated group, but in all cases of combination treatment with the peptides of Examples, the viability of cancer cells was significantly reduced even with irradiation at a tolerable dose (** p < 0.001, *** p < 0.001; right image of FIG. 16).

### Experimental Example 5: Comparison and verification of anti-cancer efficacy and effect according to the route of administration of peptides of Examples using animal models

### 5-1. Change in tumor volume in animal models injected subcutaneously with peptides of Examples

Aspc-1 cells (1 × 10⁷) were inoculated into 5-week-old BALB/c nude mice on the back flank, and the mice were divided into 15 groups: the untreated control group (control) and groups each injected subcutaneously with the peptides of Examples 15 to 28. When the tumors were grown to a volume of approximately 150 mm³, the peptides of each Example were injected subcutaneously at a dose of 20 mg/kg three times a week. The tumor size was measured with a digital caliper, and the results of changes in tumor volume were compared by the group.

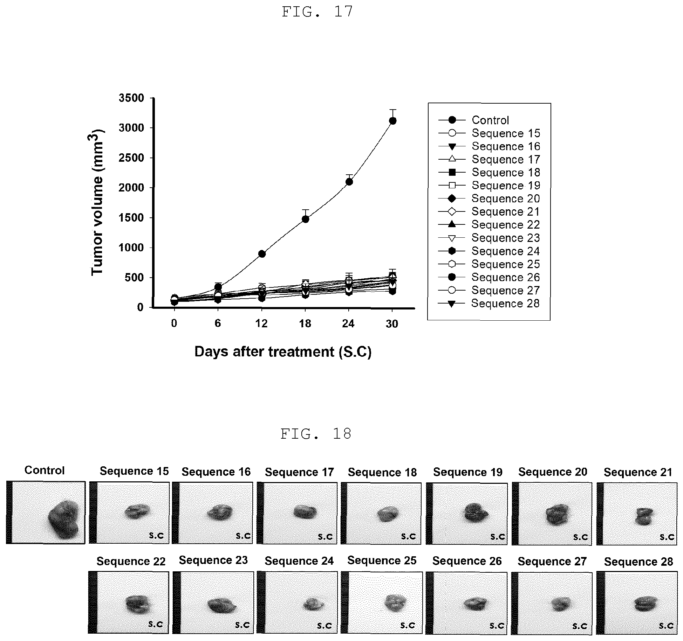

As a result, it was confirmed that after the final dose, the control group showed a large tumor growth with a final volume of about 3136 mm³, but all groups each injected subcutaneously with the peptides of Examples 15 to 28 showed significant inhibition of tumor growth (** p < 0.001; FIG. 17).

FIG. 18 shows representative tumors obtained after autopsy in all groups, and it was observed that when compared to the control group, the growth of tumor tissue was significantly inhibited in all groups injected subcutaneously with the peptides of Examples of the present disclosure.

### 5-2. Change in tumor volume in animal models injected orally with peptides of Examples

Aspc-1 cells (1 × 10⁷) were inoculated into 5-week-old BALB/c nude mice on the back flank, and the mice were divided into 15 groups: the untreated control group (control) and groups each injected orally with the peptides of Examples 1 to 14. When the tumors were grown to a volume of approximately 150 mm⁵, the peptides of each Example were injected orally at a dose of 20 mg/kg five times a week. The tumor size was measured with a digital caliper, and the results of changes in tumor volume were compared by the group.

As a result, it was confirmed that after the final dose, the control group showed a large tumor growth with a final volume of about 3517 mm³, but all groups each injected orally with the peptides of Examples 1 to 14 showed significant inhibition of tumor growth (** p < 0.001; FIG. 19).

FIG. 20 shows representative tumors obtained after autopsy in all groups, and it was observed that when compared to the control group, the growth of tumor tissue was significantly inhibited in all groups injected orally with the peptides of Examples of the present disclosure.

### Experimental Example 6: Confirmation of cytotoxicity in normal cells of peptides of Examples

Then, it was additionally confirmed whether the peptides of the present disclosure showed cytotoxicity even in normal cells. As normal cells for testing, CCD-18Co, which is a human colon fibroblast, and HDPC, which is a human dermal papilla cell, were used. Further, the peptide of SEQ ID NO: 7 was used as a representative peptide of Examples of the present disclosure.

First, 5 x 10³ cells of each normal cell were inoculated into a 96-well plate and cultured in DMEM medium for 24 hours under conditions of 37 °C and 5 % CO₂. The respective wells were divided into a group untreated with the peptide and groups treated with the peptide of SEQ ID NO: 7 (25, 50, and 100 µM, respectively), and tested.

As a result of the experiment, no significant difference was found at all between all groups treated with the peptide (24 hours, 48 hours, or 72 hours after treatment) and the untreated control group (FIGS. 21 and 22). Therefore, it was confirmed that the peptides of the Examples of the present disclosure exhibited strong anti-cancer activity in cancer cells, but showed no cytotoxicity in normal cells, and thus had a remarkably excellent effect as an anti-cancer use.

From the above description, those skilled in the art to which the present disclosure pertains will understand that the present disclosure may be embodied in other specific forms without changing the essential characteristics thereof. In this regard, it should be understood that the embodiments described above are illustrative in all respects and not restrictive.

## Claims

1. An adenosine diphosphate (ADP)-ribose binding peptide having:
any one amino acid sequence selected from the group consisting of SEQ ID NOs: 1 to 14.

2. The ADP-ribose binding peptide of claim 1, further comprising:
a cell-penetrating peptide at the N-terminus, C-terminus, or both termini.

3. The ADP-ribose binding peptide of claim 2, wherein the cell-penetrating peptide is at least one selected from the group consisting of TAT, maurocalcine, penetratin, polyarginine-derived peptides, antennapedia, transportan, VP22, and Hph-1, poly-arginine, R11(R9), Pep-1, HP4, LAH4, vetofusing-1, signal sequence-based peptides, and amphipathic peptides.

4. The ADP-ribose binding peptide of claim 2, wherein the peptide has any one amino acid sequence selected from the group consisting of SEQ ID NOs: 15 to 28.

5. A polynucleotide encoding the ADP-ribose binding peptide of any one of claims 1 to 4.

6. A vector comprising the polynucleotide of claim 5.

7. A transformant comprising the polynucleotide of claim 5.

8. A pharmaceutical composition comprising the ADP-ribose binding peptide of any one of claims 1 to 4 or a pharmaceutically acceptable salt thereof as an active ingredient, for use in preventing or treating a cancer.

9. The pharmaceutical composition for use of claim 8, wherein the cancer is at least one solid tumor selected from the group consisting of brain cancer, lung cancer, pancreatic cancer, liver cancer, breast cancer, colon cancer, kidney cancer, gastric cancer, and ovarian cancer.

10. The pharmaceutical composition for use of claim 8 or claim 9, wherein the pharmaceutical composition is administered subcutaneously or orally.

11. A pharmaceutical composition comprising an ADP-ribose binding peptide of any one of claims 1 to 4 or a pharmaceutically acceptable salt thereof as an active ingredient, for use as an anti-cancer adjuvant to enhance reactivity to a second anti-cancer drug in the treatment or prevention of a cancer.

12. The pharmaceutical composition for use of claim 11, wherein the second anti-cancer drug is a cytotoxic anti-cancer drug, a targeted anti-cancer drug or a combination thereof.

13. The pharmaceutical composition for use of claim 11 or claim 12, wherein cancer targeted by the second anti-cancer drug is at least one solid tumor selected from the group consisting of brain cancer, lung cancer, pancreatic cancer, liver cancer, breast cancer, colon cancer, kidney cancer, gastric cancer, and ovarian cancer.

14. A pharmaceutical composition comprising an ADP-ribose binding peptide of any one of claims 1 to 4 or a pharmaceutically acceptable salt thereof as an active ingredient, for use as an anti-cancer adjuvant to enhance reactivity to anti-cancer radiation therapy in the treatment or prevention of a cancer;
optionally wherein the cancer targeted by the anti-cancer radiation therapy is at least one solid tumor selected from the group consisting of brain cancer, lung cancer, pancreatic cancer, liver cancer, breast cancer, colon cancer, kidney cancer, gastric cancer, and ovarian cancer.

15. A pharmaceutical composition for preventing or treating cancer, comprising
an ADP-ribose binding peptide of any one of claims 1 to 4 or a pharmaceutically acceptable salt thereof;
optionally wherein the pharmaceutical composition further comprises a second anti-cancer drug as an active ingredient.

## Patentansprüche

1. Adenosindiphosphat(ADP)-Ribose-bindendes Peptid mit:
einer beliebigen Aminosäuresequenz, die ausgewählt ist aus der Gruppe bestehend aus SEQ ID NOs: 1 bis 14.

2. ADP-Ribose-bindendes Peptid nach Anspruch 1, ferner umfassend:
ein zellpenetrierendes Peptid an dem N-Terminus, C-Terminus oder beiden Termini.

3. ADP-Ribose-bindendes Peptid nach Anspruch 2, wobei das zellpenetrierende Peptid mindestens eines ist, das ausgewählt ist aus der Gruppe bestehend aus TAT, Maurocalcin, Penetratin, von Polyarginin abgeleiteten Peptiden, Antennapedia, Transportan, VP22 und Hph-1, Polyarginin, R11(R9), Pep-1, HP4, LAH4, Vetofusing-1, signalsequenzbasierten Peptiden und amphipathischen Peptiden.

4. ADP-Ribose-bindendes Peptid nach Anspruch 2, wobei das Peptid eine beliebige Aminosäuresequenz aufweist, die ausgewählt ist aus der Gruppe bestehend aus SEQ ID NOs: 15 bis 28.

5. Polynucleotid, das für das ADP-Ribose-bindende Peptid nach einem der Ansprüche 1 bis 4 codiert.

6. Vektor, umfassend das Polynucleotid nach Anspruch 5.

7. Transformante, umfassend das Polynucleotid nach Anspruch 5.

8. Pharmazeutische Zusammensetzung, umfassend das ADP-Ribose-bindende Peptid nach einem der Ansprüche 1 bis 4 oder ein pharmazeutisch unbedenkliches Salz davon als ein Wirkstoff zur Verwendung beim Vorbeugen oder Behandeln eines Krebses.

9. Pharmazeutische Zusammensetzung zur Verwendung nach Anspruch 8, wobei der Krebs mindestens ein solider Tumor ist, der ausgewählt ist aus der Gruppe bestehend aus Hirnkrebs, Lungenkrebs, Bauchspeicheldrüsenkrebs, Leberkrebs, Brustkrebs, Kolonkrebs, Nierenkrebs, Magenkrebs und Eierstockkrebs.

10. Pharmazeutische Zusammensetzung zur Verwendung nach Anspruch 8 oder Anspruch 9, wobei die pharmazeutische Zusammensetzung subkutan oder oral verabreicht wird.

11. Pharmazeutische Zusammensetzung, umfassend ein ADP-Ribose-bindendes Peptid nach einem der Ansprüche 1 bis 4 oder ein pharmazeutisch unbedenkliches Salz davon als ein Wirkstoff zur Verwendung als ein Antikrebsadjuvans zum Verbessern von Reaktivität gegenüber einem zweiten Antikrebsarzneimittel bei der Behandlung oder Vorbeugung eines Krebses.

12. Pharmazeutische Zusammensetzung zur Verwendung nach Anspruch 11, wobei das zweite Antikrebsarzneimittel ein zytotoxisches Antikrebsarzneimittel, ein gezieltes Antikrebsarzneimittel oder eine Kombination davon ist.

13. Pharmazeutische Zusammensetzung zur Verwendung nach Anspruch 11 oder Anspruch 12, wobei ein Krebs, auf den das zweite Antikrebsarzneimittel abzielt, mindestens ein solider Tumor ist, der ausgewählt ist aus der Gruppe bestehend aus Hirnkrebs, Lungenkrebs, Bauchspeicheldrüsenkrebs, Leberkrebs, Brustkrebs, Kolonkrebs, Nierenkrebs, Magenkrebs und Eierstockkrebs.

14. Pharmazeutische Zusammensetzung, umfassend ein ADP-Ribose-bindendes Peptid nach einem der Ansprüche 1 bis 4 oder ein pharmazeutisch unbedenkliches Salz davon als ein Wirkstoff zur Verwendung als ein Antikrebsadjuvans zum Verbessern der Reaktivität gegenüber Antikrebsstrahlentherapie bei der Behandlung oder Vorbeugung eines Krebses;
wobei optional der Krebs, auf den die Antikrebsstrahlentherapie abzielt, mindestens ein solider Tumor ist, der ausgewählt ist aus der Gruppe bestehend aus Hirnkrebs, Lungenkrebs, Bauchspeicheldrüsenkrebs, Leberkrebs, Brustkrebs, Kolonkrebs, Nierenkrebs, Magenkrebs und Eierstockkrebs.

15. Pharmazeutische Zusammensetzung zum Vorbeugen oder Behandeln von Krebs, umfassend
ein ADP-Ribose-bindendes Peptid nach einem der Ansprüche 1 bis 4 oder ein pharmazeutisch unbedenkliches Salz davon;
wobei optional die pharmazeutische Zusammensetzung ferner ein zweites Antikrebsarzneimittel als einen Wirkstoff umfasst.

## Revendications

1. Peptide de liaison à l'adénosine diphosphate (ADP)-ribose présentant :
une quelconque séquence d'acides aminés choisie dans le groupe constitué par les SEQ ID n° : 1 à 14.

2. Peptide de liaison à l'ADP-ribose selon la revendication 1, comprenant en outre :
un peptide de pénétration cellulaire au niveau de l'extrémité N-terminale, de l'extrémité C-terminale ou des deux extrémités.

3. Peptide de liaison à l'ADP-ribose selon la revendication 2, ledit peptide de pénétration cellulaire étant au moins l'un choisi dans le groupe constitué par la TAT, la maurocalcine, la pénétratine, les peptides dérivés de polyarginine, l'antennapedia, le transportan, la VP22 et la Hph-1, la poly-arginine, le R11 (R9), le Pep-1, la HP4, le LAH4, le vetofusing-1, les peptides à base de séquences signal et les peptides amphipathiques.

4. Peptide de liaison à l'ADP-ribose selon la revendication 2, ledit peptide présentant une quelconque séquence d'acides aminés choisie dans le groupe constitué par les SEQ ID n° : 15 à 28.

5. Polynucléotide codant le peptide de liaison à l'ADP-ribose selon l'une quelconque des revendications 1 à 4.

6. Vecteur comprenant le polynucléotide selon la revendication 5.

7. Transformant comprenant le polynucléotide selon la revendication 5.

8. Composition pharmaceutique comprenant le peptide de liaison à l'ADP-ribose selon l'une quelconque des revendications 1 à 4 ou un sel pharmaceutiquement acceptable de celui-ci en tant qu'ingrédient actif, destinée à être utilisée dans la prévention ou le traitement d'un cancer.

9. Composition pharmaceutique destinée à être utilisée selon la revendication 8, ledit cancer étant au moins une tumeur solide choisie dans le groupe constitué par le cancer du cerveau, le cancer du poumon, le cancer du pancréas, le cancer du foie, le cancer du sein, le cancer du côlon, le cancer du rein, le cancer de l'estomac et le cancer de l'ovaire.

10. Composition pharmaceutique destinée à être utilisée selon la revendication 8 ou la revendication 9, ladite composition pharmaceutique étant administrée par voie sous-cutanée ou par voie orale.

11. Composition pharmaceutique comprenant un peptide de liaison à l'ADP-ribose selon l'une quelconque des revendications 1 à 4 ou un sel pharmaceutiquement acceptable de celui-ci en tant qu'ingrédient actif, destinée à être utilisée en tant qu'adjuvant anticancéreux pour améliorer la réactivité à un second médicament anticancéreux dans le traitement ou la prévention d'un cancer.

12. Composition pharmaceutique destinée à être utilisée selon la revendication 11, ledit second médicament anticancéreux étant un médicament anticancéreux cytotoxique, un médicament anticancéreux ciblé ou une combinaison de ceux-ci.

13. Composition pharmaceutique destinée à être utilisée selon la revendication 11 ou la revendication 12, ledit cancer ciblé par le second médicament anticancéreux étant au moins une tumeur solide choisie dans le groupe constitué par le cancer du cerveau, le cancer du poumon, le cancer du pancréas, le cancer du foie, le cancer du sein, le cancer du côlon, le cancer du rein, le cancer de l'estomac et le cancer de l'ovaire.

14. Composition pharmaceutique comprenant un peptide de liaison à l'ADP-ribose selon l'une quelconque des revendications 1 à 4 ou un sel pharmaceutiquement acceptable de celui-ci en tant qu'ingrédient actif, destinée à être utilisée en tant qu'adjuvant anticancéreux pour améliorer la réactivité à une radiothérapie anticancéreuse dans le traitement ou la prévention d'un cancer ;
éventuellement, ledit cancer ciblé par la radiothérapie anticancéreuse étant au moins une tumeur solide choisie dans le groupe constitué par le cancer du cerveau, le cancer du poumon, le cancer du pancréas, le cancer du foie, le cancer du sein, le cancer du côlon, le cancer du rein, le cancer de l'estomac et le cancer de l'ovaire.

15. Composition pharmaceutique destinée à la prévention ou au traitement d'un cancer, comprenant
un peptide de liaison à l'ADP-ribose selon l'une quelconque des revendications 1 à 4, ou un sel pharmaceutiquement acceptable de celui-ci ;
éventuellement, ladite composition pharmaceutique comprenant en outre un second médicament anticancéreux en tant qu'ingrédient actif.
